Europäisches Patentamt

European Patent Office

Office européen des brevets

(11) Publication number: **0 042 703**

**B1**

(12) **EUROPEAN PATENT SPECIFICATION**

(45) Date of publication of patent specification: **28.11.84**

(21) Application number: **81302619.2**

(22) Date of filing: **12.06.81**

(51) Int. Cl.³: **A 61 M 25/00**

(54) **Self-guiding type medical vascular catheter.**

(30) Priority: **23.06.80 JP 83955/80**

(43) Date of publication of application:
**30.12.81 Bulletin 81/52**

(45) Publication of the grant of the patent:
**28.11.84 Bulletin 84/48**

(84) Designated Contracting States:
**AT BE CH DE FR GB IT LI LU NL SE**

(56) References cited:
**EP-A-0 014 424
DE-A-2 927 788
DE-C- 94 003
FR-A-1 278 426
US-A-2 268 321
US-A-3 434 869
US-A-3 687 142
US-A-3 983 879**

(73) Proprietor: **TORAY MONOFILAMENT
COMPANY LIMITED
1, Aza Kawara, Showa-cho
Okazaki-shi, Aichi-ken (JP)**

(73) Proprietor: **OSAKA CITY GOVERNMENT
3-20, Nakanoshima 1-chome
Kita-ku, Osaka 530 (JP)**

(72) Inventor: **Igawa, Keisuke
6-27, Kajita-cho
Obu-shi Aichi-ken (JP)**
Inventor: **Yamada, Kunio
15, Nakanokiri Choda-cho
Nishio-shi Aichi-ken (JP)**
Inventor: **Yamada, Ryusaku
1-4-19 Nishiyamadai Sayama-cho
Minamikawa-chi-gun Osaka (JP)**
Inventor: **Kudo, Hiroaki
227-2, Higashishigigaoka Sango-cho
Ikoma-gun Nara-ken (JP)**

(74) Representative: **Ellis, John Clifford Holgate et al
MEWBURN ELLIS & CO. 2/3 Cursitor Street
London EC4A 1BQ (GB)**

## Description

This invention relates to a self-guiding type multi-purpose medical vascular catheter, viz, a medical vascular catheter capable of guiding its tip by itself to peripheral blood vessels in the affected parts of a living body for withdrawal of blood and other specimens from the affected parts, or for infusion of a drug solution to the affected parts.

A medical vascular guide wire has a function for aiding the insertion of a catheter into peripheral blood vessels in the affected parts. When the guide wire is used in combination with the catheter, first, the guide wire is inserted into the blood vessels. When the guide wire reaches the peripheral blood vessels in affected parts, the catheter is inserted into the blood vessels so that the catheter sheathes the guide wire and then, the guide wire is withdrawn therefrom leaving the catheter. Thereafter, a drug solution is infused through the catheter to the affected parts.

Most conventional guide wires are coil-form metal wires, such as stainless steel wires, supplied by, for example, COOK Inc., Universal Medical Instrument Corp. and USCI, a division of C.R. Bard, Inc. Most conventional catheters are made of synthetic resin tubes and supplied by, for example, Universal Medical Instrument Corp., Electro-Catheter Corp. and USCI, a division of C.R. Bard, Inc.

The above-mentioned coiled metal guide wires have some disadvantages. Firstly, the coiled metal wires possess an uneven surface and hence thrombi are liable to be deposited thereon. Secondly, the metal wires are too rigid to permit their tips to reach the blood vessels in the affected parts without causing undue strain or pain to the patient, or taking a great deal of time. Thirdly, the coiled metal wires are not easy to manipulate so that it is sometimes difficult for their tips to reach the affected parts. Fourthly, there is a danger that the coiled metal wires may injure the intimae of blood vessels. The above-mentioned catheters also have some disadvantages. Firstly, the catheters are larger in diameter than the guide wires and therefore, it is difficult for the catheter tip to reach the intended location, particularly in peripheral blood vessels, where the guide wire has reached. Furthermore, it is generally difficult to accomplish the catheterization without causing undue strain or pain to the patient. Secondly, in the process of catheterization, many kinds of catheters and guide wires must be used in combination. Thus the procedure requires a substantially long period of time.

A primary object of the present invention is to provide a self-guiding type medical vascular catheter capable of guiding its tip by itself to the peripheral blood vessels, which can be easily and freely manipulated for permitting its tip part to reach the peripheral blood vessels without restraint.

Another object of the present invention is to provide a self-guiding type medical vascular catheter having a smooth surface on which thrombi are deposited only to a slight extent, and which catheter is sufficiently flexible for permitting its tip part to reach peripheral blood vessels in the affected parts without undue strain to the patient or taking a great deal of time and further without injury to the intimae of blood vessels.

Other objects and advantages of the present invention will be apparent from the following description.

US—A—3 687 142 describes a catheter comprising a tip part connected by a flexible thread of smaller diameter to a flexible tube, all of which are capable of being passed through a catheter tube by manual operation of a stylet wire. The tip part or embolus may be of metal. In one embodiment the embolus, thread and tube are formed as an integral whole, the embolus being hollow and containing a liquid contrast medium. In order to use the catheter, the catheter tube is manipulated into the region of the tract to be catheterized, whereupon the flexible tube is advanced through the catheter tube by means of the stylet until it fully projects therefrom, and then the catheter is directed into the tract by manipulation of the catheter tube or the wire, as detailed more fully in the specification of that patent.

The catheter of the present invention likewise comprises a tip part containing material opaque to X-rays, a relatively flexible part and a manipulating part arranged in that order, at least the manipulating part having a hollow therealong to allow fluid to be passed through the catheter to an outlet opening at or in close proximity its front end. The catheter of the invention is characterised in that (i) the tip part, the relatively flexible part and the manipulating part are made from a single monofilament of synthetic resin, the manipulating part leading into the relatively flexible part through a tapering part of the filament (ii) the X-ray opaque material in the tip part consists of an X-ray impermeable metal embedded therein and (iii) said hollow has an X-ray impermeable metal inserted therein at least in the manipulating part, the hollow having a diameter larger than that of the X-ray impermeable metal inserted therein so as to leave a lumen for the liquid and/or gas to pass therethrough, which lumen is opened to the outside of the body through an inlet opening at the distal end of the manipulating part.

In the drawings:

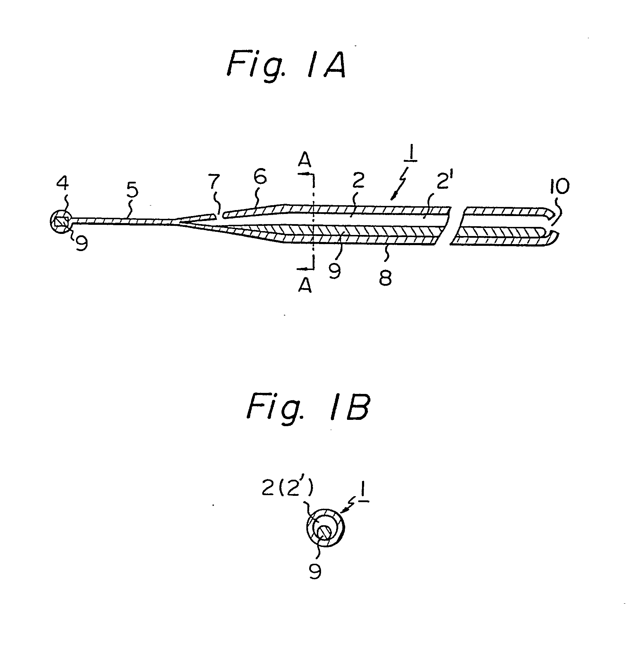

Fig. 1A is an enlarged longitudinal sectional view illustrating one example of a self-guiding type catheter of the invention; and

Fig. 1B is an enlarged transverse sectional view taken along the line A—A in Fig. 1.

Referring to Figs. 1A and 1B, self-guiding type catheter 1 is made of a synthetic resin monofilament having a hollow 2 extending along the monofilament axis. The catheter 1 is composed of a tip part 4, a relatively flexible part 5 (which is hereinafter referred to as "flexible part" for brevity), a tapering part 6 and a manipulating part 8. These four parts continuously form a line, in that order, along the monofilament axis.

The tip part 4 is of a hollow structure and has X-ray impermeable metal 9 inserted therein. The shape of the tip part 4 is not particularly limited but may preferably be spherical, oval spherical or end-rounded cylindrical. The size of the tip part 4 is preferably about 0.5—3 mm, more preferably about 0.7—2 mm, in maximum diameter, and preferably about 0.5—5 mm, more preferably about 0.7—4 mm, in length.

The flexible part 5 may be either hollow or solid and possesses a substantially uniform diameter over its entire length. The size of the flexible part 5 is preferably about 0.1—0.5 mm, more preferably about 0.15—0.4 mm, in diameter, and preferably about 10—100 mm, more preferably about 20—70 mm, in length. The flexible part 5 possesses a smaller diameter, and is more flexible and pliable than the other parts, and furthermore, is sufficiently strong to prevent the catheter from being broken between the tip part 4 and the flexible part 5.

The tapering part 6 tapers from its end adjacent to the manipulating part 8 toward the flexible part 5. The size of the tapering part 6 is preferably about 10—100 mm, more preferably about 20—70 mm, in length. Although there may be no clear demarcation between the tapering part 6 and the flexible part 5, the total length of these two parts should preferably be not greater than 200 mm. The tapering part 6 may be either hollow or solid. It is, however, preferable that the tapering part 6 be hollow and have an X-ray impermeable metal inserted therein.

The manipulating part 8 is of a hollow monofilament structure. Its diameter is preferably about 0.7—3 mm, more preferably about 0.7—2 mm. Its length should be at least about 200 mm, preferably 50—200 cm and more preferably 70—150 cm. The manipulating part 8 has an X-ray impermeable metal 9, in the form of a wire of about 0.2—0.4 mm in diameter, inserted in the hollow thereof. Therefore, the manipulating part 8 possesses not only a high stiffness but also a suitable degree of rigidity as well as a suitable degree of flexibility and elasticity, and this part of the catheter can be easily and freely manipulated for permitting the tip part of the catheter to reach the peripheral blood vessels without restraint.

The above-mentioned numerical values for the size of the self-guiding type catheter are mere exemplifications and it should be understood that the size of the catheter is not particularly limited to such values. These sizes may be suitably varied depending upon, for example, the age and physical condition of the patient or the state of the parts for which treatment is intended.

The body of the self-guiding type catheter 1 has a hollow 2 extending at least from one end of the manipulating part 8 to the tapering part 6 or to the other end of the manipulating part 8 or to a point in close proximity to said other end of the manipulating part. The hollow 2 has a diameter larger than that of an X-ray impermeable metal 9 inserted therein over substantially the entire length of the hollow, and therefore, a lumen defined by a space 2' is formed within the hollow 2. In general, it is preferable that the diameter of the hollow 2 is as large as possible. This space 2' forms a lumen for permitting a liquid and/or a gas, such as a drug solution, to pass therethrough. The lumen is opened to the outside of the body of the catheter 1 through an inlet opening 10 at the distal end of the lumen and through an outlet opening 7. The outlet opening 7 is bored preferably in the tapering part 6 as illustrated in Fig. 1A. However, in the case where the flexible part 5 is hollow and has a relatively large diameter, the outlet opening 7 may be bored in the flexible part 5 or the tip part 4. The outlet opening 7 preferably has a sectional shape, as shown in Fig. 1A, such that the section becomes broader toward the outside of the catheter body so as to avoid the undesirable retention of a drug solution. The lumen defined by the space 2' is usually sterilized.

The X-ray impermeable (or radio-opaque) metal is inserted at least in the tip part and the manipulating part of the catheter, so that the catheter can be monitored from the outside of the patient's body during the introduction of the catheter into blood vessels. The X-ray impermeable metal 9 inserted in the hollow 2 preferably possesses a diameter corresponding to 20—80% of the diameter of the hollow 2. As the X-ray impermeable metal, a tungsten wire is preferably used because of its high radio-opacity. In view of the possible reactivity of the X-ray impermeable metal with a drug solution, it is preferable that the X-ray impermable metal is coated with a resin such as polyethylene, polyethylene terephthalate or a fluorine-containing resin. Furthermore, the X-ray impermeable metal wire is preferably tapered at one end portion thereof toward the tip part 4. The tapered metal wire exhibits an enhanced flexibility, as compared with a non-tapered metal wire, and thus, the catheter can be more unrestrictedly moved in blood vessels and more easily manipulated. Moreover, the tapered metal wire permits a drug solution to pass through the lumen more smoothly.

The inlet opening 10 at the distal end of the lumen preferably has a diameter smaller than that of the X-ray impermeable metal wire 9 so that the metal wire 9 does not come out through the inlet opening 10. The inlet opening 10 may be capped when the catheter is not applied. Furthermore, the end portion of the monofilament body, having the inlet opening 10, may preferably be of a structure such that a connector or an injector can be connected thereto.

The multi-purpose medical vascular catheter of the invention may be made of any melt-extrudable thermoplastic synthetic resins. The thermoplastic synthetic resins used include, for example, polyesters, such as polyethylene terephthalate and polybutylene terephthalate; polyamides, such as nylon-6, nylon-66, nylon-610, nylon-11, nylon-12, and nylon-6/66; polyolefins, such as polyethylene and polypropylene; and, fluorine-containing resins, such as copolymer of tetrafluoroethylene with hexafluoropropylene. These thermoplastic synthetic resins may be used either alone or in combination.

The medical vascular catheter of the invention may be manufactured as follows. A thermoplastic synthetic resin is melt-extruded through an annular orifice of a spinneret provided in an extruder, to obtain an undrawn or slightly drawn continuous hollow monofilament. The continuous hollow monofilament is cut into predetermined lengths. A limited portion of the entire length of each hollow monofilament, which portion is adjacent to one end portion thereof, is drawn several times its original length, whereby a relatively flexible part 5 and a tapering part 6 are simultaneously formed. X-ray impermeable metal 9 is inserted into the hollow of the undrawn or slightly drawn end portion thereof and then, its open end is heat-sealed to form the tip part 4. X-ray impermeable metal 9 is also inserted into the continuously extending hollow of the manipulating part 8 from the end thereof opposite to the tip part 4 and then the open end is heated so that the inlet opening is narrowed. As hereinbefore mentioned, the X-ray impermeable metal wire is tapered at one end thereof prior to its insertion into the hollow of the monofilament body. The tapered metal wire exhibits an enhanced flexibility and the catheter can be more unrestrictedly moved in blood vessels and more easily manipulated. Then, an outlet opening 7 (Fig. 1A) is drilled through the wall of the tapering part by using a fine drill. The end portion of the monofilament body, which has an inlet opening 10 (Fig. 1A), which may be provided with a cap or an adaptor to connect the catheter with an infusion pump.

The hollow monofilament body of the catheter of the invention can be surface-treated in order to enhance its self-lubricating property and thus, to minimize the thrombus deposition and to make the manipulation of the catheter very easy. The surface treatment employed includes (i) a silicon treatment, (ii) a fluorine-containing resin coating treatment or (iii) a plasma spark discharge treatment. These surface treatments can be effected as follows.

(i) Silicon coating treatment

A hollow monofilament body is passed several times through a bath of a solution of a medical silicone dissolved in, for example, isopropanol, and then the so coated monofilament body is dried under reduced pressure, thereby removing the solvent therefrom. Prior to the silicone coating treatment, the monofilament body may be surface-treated with sandpaper in order to facilitate the silicone coating treatment.

(ii) Fluorine-containing resin coating treatment

A hollow monofilament body is treated with a solution of a fluorine-containing resin, such as "Teflon 30 J" (trade name an aqeuous suspension containing about 60 weight % of finely divided polytetrafluoroethylene having a particle size of about 0.3 micron, supplied by Mitsui Fluorochemical Co.) and "Teflon D-1" (trade name, an aqueous suspension of finely divided polytetrafluoroethylene, supplied by Daikin Kogyo K.K.). The treating procedure may be similar to that explained above with regard to the silicone coating treatment.

(iii) Plasma spark discharge treatment

A hollow monofilament body is placed in a chamber which is maintained at a reduced pressure of below about 1 mmHg and into which a small amount of a gaseous organic fluorocompound is introduced. Then, a plasma spark discharge is effected, thereby introducing fluorine substituents into the polymer forming the surface of the monofilament.

The advantages of the self-guiding type medical vascular catheter will be explained. When the catheter is inserted in blood vessels, the tip part 4 is capable of drifting in the blood stream and thus, freely moving in blood vessels in response to delicate manipulation even in the case where the blood vessels wind and curve. The tip part does not penetrate into intimae of blood vessels. Thus, the tip part 4 reaches the affected parts without even a slight strain being caused on the patient, and there is no risk of injuring the intimae of blood vessels. This is in a striking contrast to conventional metal guide wires, which must be forced into blood vessels.

As the tip part 4 has an X-ray impermeable metal inserted in the hollow thereof, the tip part 4 can be easily monitored by an X-ray projection.

The flexible part 5 permits the free movement of the tip part 4 in blood vessels. The tapering part 6 can prevent unusual retention of blood and also, ensures the unrestricted manipulation of the catheter. Thus, a highly skilled and delicate manipulation of the catheter can be easily effected, which manipulation is required for advancing the catheter into the intended blood vessel branch at a turning point. The flexibility and pliability of the tapering part 6 are enhanced by the fact that one end portion of the X-ray permeable metal inserted in the taper part 6 or the extended hollow part from tapering part 6 to manipulating part 8 is formed taperingly. Aforesaid tip part 4, flexible part 5 and tapering part 6 render the catheter so flexible and pliable as not to penetrate into even bean curd. Therefore, the injury of the intimae of blood vessels and undue strain on the patient can be remarkably reduced.

The manipulating part 8 which is made of the synthetic resin monofilament has a smooth surface and therefore, thrombi are deposited thereon only to a slight extent. Furthermore, the manipulating part 8 has an X-ray impermeable metal inserted therein through its entire length; therefore, this part is not only easily manipulated but also can be monitored easily.

After the tip part of the catheter has been guided to the intended affected parts, if a drug solution is introduced and supplied under pressure from the inlet opening 10 on the rear end of the lumen 2', the drug solution can effectively be supplied from the outlet opening 7 to the affected parts and a high medical effect can be attained. Furthermore, since the drug solution is selectively supplied to the affected parts alone, the intended medical treatment can be accomplished with use of a reduced amount of the drug solution and a risk of side effects can be reduced.

The self-guiding type catheter of the present invention can act both as a guide wire and as a catheter. Accordingly, the medical treatment implement can be simplified and the treatment time can be shortened.

The catheter of the present invention is very valuable for medical treatment utilizing the ultraselective catheterizing technique. The catheter can be used for sampling blood from the affected parts. It can also be used for monitoring the blood pressure by intruding a liquid or gas into the catheter. Furthermore, the catheter can be used as a so-called indwelling catheter which is inserted in a blood vessel and left therein for intermittent infusion of a drug solution.

The invention will now be illustrated by the following example.

Example

A hollow monofilament having a diameter of 0.8 mm and a hollow diameter of 0.35 mm was prepared from polyethylene terephthalate according to the conventional melt-spinning method using a ring spinneret, and the hollow monofilament was cut into a length of 1.5 m. One end portion of the cut monofilament was locally drawn at a draw ratio of about 3 except a part of about 3 mm to be formed into a spherical tip part, whereby a flexible part having a diameter of 0.3 mm and a length of 50 mm and a tapering part having a length of 30 mm were formed simultaneously. A tungsten piece having a diameter of 0.3 mm and a length of 1 mm was inserted into the top end part to be formed into a spherical tip part and the open hole was melt-sealed. A top-tapered tungsten wire having a diameter of 0.25 mm and length of 1.3 m was inserted from the rear end into the hollow portion of the tapering part of the catheter, in a manner such that the tapered top part of the tungsten wire was directed towards the spherical tip part. Then, the rear end portion was heated so that the opening size was reduced to obtain a catheter having a hollow extending from the rear end to the tapering part and which has the tungsten wire inserted therein. A small opening having a diameter of 0.25 mm was formed from the outside on the shoulder of the tapered part whereby a drug solution discharge opening was formed.

The so obtained catheter having a structure as shown in Fig. 1 was used in clinical examination in which the catheter was advanced from the abdominal aorta through the coeliac artery to the hepatic artery. Both the images of the spherical part and the tapering part produced under irradiation with X-rays were clear and their positions could easily be monitored. The top end portion of the catheter could easily be guided to the intended affected part of the liver only by handling the manipulating part. When a drug solution was supplied into the lumen in advance and the solution was forced by a precision metering pump connected to the drug solution inlet opening on the rear end, the drug solution could be supplied selectively to the affected part. This medical treatment required only about 1/5 of the treatment time necessary in the conventional method using a metal guide wire and a catheter in combination, and the pain given to a patient could remarkably be reduced. After the treatment, the catheter could easily be withdrawn, and the withdrawn catheter could easily be cleaned by wiping it with gauze and deposition of thrombi was hardly observed.

**Claims**

1. A self-guiding type medical vascular catheter comprising a tip part (4) containing material (9) opaque to X-rays, a relatively flexible part (5) having a smaller diameter than the other parts and a manipulating part (8) arranged in that order, at least the manipulating part having a hollow (2) therealong to allow fluid to be passed through the catheter to an outlet opening (7) at or in close proximity to its front end, characterised in that (i) the tip part (4), the relatively flexible part (5) and the manipulating part (8) are made from a single monofilament of synthetic resin, the manipulating part leading into the relatively flexible part (5) through a tapering part (6) of the filament (ii) the X-ray opaque material (9) in the tip part consists of an X-ray impermeable metal embedded therein and (iii) said hollow (2) has an X-ray impermeable metal inserted therein at least in the manipulating part, the hollow having a diameter larger than that of the X-ray impermeable metal inserted therein so as to leave a lumen for the liquid and/or gas to pass therethrough, which lumen is opened to the outside of the body through an inlet opening (10) at the distal end of the manipulating part.

2. A self-guiding type medical vascular catheter according to claim 1, wherein the X-ray impermeable metal in the manipulating part is in the form of a wire, one end portion of which is tapered and which wire is inserted in said hollow in a manner such that the tapered end thereof is directed towards the tip part of the monofilament body.

3. A self-guiding type medical vascular catheter according to claim 1 or claim 2, wherein the hollow monofilament is made of at least one melt-extrudable thermoplastic synthetic resin selected from the group consisting of polyesters, polyamides and polyolefins.

4. A self-guiding type medical vascular catheter according to any one of claims 1 to 3, wherein the catheter body has been surface-treated by (i) a silicon coating treatment, (ii) a fluorine-containing resin coating treatment, or (iii) a plasma spark discharge treatment effected in an atmosphere of a gaseous organic fluorine-containing compound.

5. A self-guiding type medical vascular catheter according to any one of the preceding claims, wherein the respective parts of the monofilament body have the dimensions:

tip part: diameter is about 0.5—3 mm and length is about 0.5—5 mm;

relatively flexible part: diameter is about 0.1—0.5 mm and length is about 10—100 mm;

tapered part: length is about 10—100 mm, and

manipulating part: diameter is about 0.7—3 mm and length is at least about 200 mm.

6. A self guiding type medical vascular catheter according to claim 5, wherein the X-ray impermeable metal in the manipulating part (8) is in the form of a wire of diameter about 0.20 mm and 0.40 mm.

7. A self-guiding type medical vascular catheter according to any one of the preceding claims, wherein the X-ray impermeable metal in the manipulating part (8) has a diameter corresponding to 20 to 80% of the diameter of the hollow.

**Revendications**

1. Cathéter médical endo-veineux du type autoguidé, comprenant une partie de pointe (4) qui contient une matière (9) opaque aux rayons X, une partie relativement flexible (5) ayant un plus petit diamètre que les autres parties, et une partie de manipulation (8), disposées dans cet ordre, au moins la partie de manipulation présentant une cavité (2) s'étendant sur sa longueur pour permettre de faire passer un fluide à travers le cathéter jusqu'à une ouverture de sortie (7) située à ou très proche de son extrémité avant, caractérisé en ce que (i) la partie de pointe (4), la partie relativement flexible (5) et la partie de manipulation (8) sont faites à partir d'un unique monofilament de résine synthétique, la partie de manipulation menant à la partie relativement flexible (5) en passant par une partie (6) à section décroissante du filament, (ii) la matière (9) opaque aux rayons X contenue dans la partie de pointe consiste en un métal imperméable aux rayons X qui y est noyé et (iii) ladite cavité (2) présente un métal imperméable aux rayons X qui y est inséré, au moins dans la partie de manipulation, la cavité possédant un diamètre supérieur à celui du métal imperméable aux rayons X qui y est inséré, de manière à laisser libre un conduit pour le passage du liquide et/ou du gaz à travers cette lumière, laquelle lumière est ouverte sur l'extérieur du corps, à travers une ouverture d'entrée (10), à l'extrémité distale de la partie de manipulation.

2. Cathéter médical endo-veineux du type autoguidé selon la revendication 1, dans lequel le métal imperméable aux rayons X contenu dans la partie de manipulation se présente sous la forme d'un fil dont une partie terminale est effilée, et lequel fil est inséré dans ladite cavité de telle manière que son extrémité effilée soit dirigée vers la partie de pointe du corps en monofilament.

3. Cathéter médical endo-veineux du type autoguidé selon la revendication 1 ou la revendication 2, dans lequel le monofilament creux est fait d'au moins une résine synthétique thermoplastique pouvant être extrudée par fusion, choisie dans la groupe composé des polyesters, des polyamides et des polyoléfines.

4. Cathéter médical endo-veineux du type autoguidé selon l'une quelconque des revendications 1 à 3, dans lequel le corps du cathéter a été traité en surface par (i) un traitement de revêtement au silicone, (ii) un traitement de revêtement par une résine contenant du fluor, (iii) un traitement par décharge disruptive dans un plasma effectué dans une atmosphère d'un composé organique gazeux contenant du fluor.

5. Un cathéter médical endo-veineux du type auto-guidé selon l'une quelconque des revendications précédentes, dans lequel les parties respectives du corps en monofilament possèdent les dimensions suivantes:

partie de pointe: le diamètre est d'environ 0,5 à 3 mm et la longeur est d'environ 0,5 à 5 mm;

partie relativement flexible: le diamère est d'environ 0,1 à 0,5 mm et la longueur est d'environ 10 à 100 mm;

partie à section décroissante: la longueur est d'environ 10 à 100 mm; et

partie de manipulation: le diamètre est d'environ 0,7 à 3 mm et la longueur est au moins d'environ 200 mm.

6. Un cathéter médical endo-veineux du type auto-guidé selon la revendication 5, dans lequel le métal imperméable aux rayons X contenu

**0 042 703**

dans la partie de maniulation (8) est présenté sous la forme d'un fil d'un diamètre d'environ 0,20 à 0,40 mm.

7. Un cathéter médical endo-veineux du type auto-guidé selon l'une quelconque des revendications précédentes, dans lequel le métal imperméable aux rayons X contenu dans la partie de manipulation (8) posséde un diamètre correspondant à 20 à 80% du diamètre de la cavité.

## Patentansprüche

1. Selbstführender medizinischer Gefäßkatheter mit einem Spitzenteil (4), der eine gegen Röntgenstrahlen undurchlässiges Material (9) enthält, einem relativ biegsamem Teil (5) mit geringerem Durchmesser als die anderen Teile und einem Handhabungsteil (8), welche Teile in der genannten Reihenfolge angeordnet sind, wobei wenigstens der Handhabungsteil in seiner Längsrichtung von einem Hohlraum (2) durchsetzt ist, um das durch den Katheter zu führende Fluid zu einer an seinem Stirnende oder in unmittelbarer Nähe seines Stirnendes befindlichen Auslaßöffnung (7) zu leiten, dadurch gekennzeichnet, daß (i) der Spitzenteil (4), der relativ biegsame Teil (5) und der Handhabungsteil (8) aus einem Einzelstrang aus Kunstharz gefertigt sind und der Handhabungsteil in den relativ biegsamen Teil (5) über einen verjüngten Teil (6) des Stranges übergeht, (ii) das gegen Röntgenstrahlen undurchlässige Material (9) im Spitzenteil aus einem darin eingebetteten, gegen Röntgenstrahlen undurchlässigen Metall besteht und (iii) in den Hohlraum (2) wenigstens im Bereich des Handhabungsteiles ein gegen Röntgenstrahlen undurchlässiges Metall eingesetzt ist, wobei der Hohlraum einen größeren Durchmesser als der des in ihn eingesetzen, gegen Röntgenstrahlen undurchlässigen Metalls besitzt, so daß für den Durchtritt der Flüssigkeit und/oder des Gases ein Kanal freigelassen wird, der sich gegen die Außenseite des Körpers über eine Einlaßöffnung (10) am Vorderende des Handhabungsteiles öffnet.

2. Gefäßkatheter nach Anspruch 1, dadurch gekennzeichnet, daß das gegen Röntgenstrahlen undurchlässige Metall im Handhabungsteil in Form eines Drahtes vorliegt, von dem ein Ende verjüngt ist und der in den genannten Hohlraum derart eingesetzt ist, daß das verjüngte Ende gegen den Spitzenteil des Einzelstrangkörpers weist.

3. Gefäßkatheter nach Anspruch 1 oder 2, dadurch gekennzeichnet, daß der hohle Einezelstrang aus wenigstens einem schmelzextrudierbaren thermoplastischen Kunsthartz der Gruppe umfassened Polyester, Polyamide und Polyolefine hergestellt ist.

4. Gefäßkatheter nach einem der Ansprüche 1 bis 3, dadurch gekennzeichnet, daß der Katheterkörper mit (i) einer Silicon-Beschichtungsbehandlung, (ii) einer Beschichtungsbehandlung mit einem fluorhaltigen Harz oder (iii) einer Plasmafunkenentladungsbehandlung, durchgeführt in einer Atmosphäre einer gasförmigen fluorhaltigen Verbindung, vorbehandelt wurde.

5. Gefäßkatheter nach einem der vorhergehenden Ansprüche, dadurch gekennzeichnet, daß die jeweiligen Teile des Einzelstrangkörpers folgende Abmessungen besitzen:
Spitzenteil: Durchmesser etwa 0,5—3 mm, Länge etwa 0,5—5 mm;
relativ biegsamer Teil: Durchmesser etwa 0,1—0,5 mm, Länge etwa 10—100 mm;
verjüngter Teil: Länge etwa 10—100 mm; und
Handhabungsteil: Durchmesser etwa 0,7—3 mm, Länge wenigstens etwa 200 mm.

6. Gefäßkatheter nach Anspruch 5, dadurch gekennzeichnet, daß das gegen Röntgenstrahlen undurchlässige Metall im Handhabungsteil (8) in Form eines Drahtes mit einem Durchmesser von etwa 0,20 bis 0,40 mm vorliegt.

7. Gefäßkatheter nach einem der vorhergehenden Ansprüche, dadurch gekennzeichnet, daß das gegen Röntgenstrahlen undurchlässige Metall im Handhabungsteil (8) einen Durchmesser entsprechend 20 bis 80% des Durchmessers des Hohlraumes besitzt.

# Fig. 1A

# Fig. 1B

2(2')  1

9